# EUROPEAN PATENT SPECIFICATION

(11) **EP 2 026 749 B1**
(45) Date of publication and mention of the grant of the patent: **02.11.2016**
(21) Application number: 07777125.1
(22) Date of filing: 17.05.2007
(51) Int. Cl.: A61K 8/33, A61K 8/34, A61K 8/36, A61K 8/37, A61K 8/41, A61K 8/892, A61K 8/895, A61K 8/898, A61Q 5/02, A61Q 5/12

(54) **HAIR CARE COMPOSITION COMPRISING AN AMINOSILICONE AND A HIGH VISCOSITY SILICONE COPOLYMER EMULSION**
HAARPFLEGEZUSAMMENSETZUNG MIT EINEM AMINOSILIKON UND EINER COPOLYMER-EMULSION VON HOHER VISKOSITÄT
PRÉPARATION DE SOIN CAPILLAIRE COMPRENANT UNE AMINOSILICONE ET UNE ÉMULSION DE COPOLYMÈRE SILICONE DE VISCOSITÉ ÉLEVÉE

(30) Priority: 17.05.2006 US 800990 P
(43) Date of publication of application: 25.02.2009
(73) Proprietor: THE PROCTER & GAMBLE COMPANY, Cincinnati, OH 45202 (US)
(72) Inventor: TORGERSON, Peter, Marte, Washington Court House, Ohio 43160 (US); UEHARA, Nobuaki, Kobe, Hyogo 658-0082 (JP)
(74) Representative: Hoyng Rokh Monegier LLP
(86) International application number: PCT/US2007/011841
(87) International publication number: WO 2007/136708

(56) References cited:
- WO-A-03/047544
- WO-A-2006/045418
- WO-A-2006/138201
- WO-A1-03/101411
- WO-A1-2006/044959

## Description

### FIELD

The present invention relates to hair care compositions containing an aminosilicone and a high viscosity silicone copolymer emulsion.

### BACKGROUND

Human hair becomes soiled due to its contact with the surrounding environment and from the sebum secreted by the scalp. The soiling of hair causes it to have a dirty feel and an unattractive appearance. The soiling of the hair necessitates shampooing with frequent regularity.

Shampooing cleans the hair by removing excess soil and sebum. However, shampooing can leave the hair in a wet, tangled, and generally unmanageable state. Once the hair dries, it is often left in a dry, rough, lusterless, or frizzy condition due to removal of the hair's natural oils and other natural conditioning and moisturizing components. The hair can further be left with increased levels of static upon drying, which can interfere with combing and result in a condition commonly referred to as "fly-away hair," or contribute to an undesirable phenomenon of "split ends." Further, chemical treatments, such as perming, bleaching, or coloring hair, can also damage hair and leave it dry, rough, lusterless, and damaged.

A variety of approaches have been developed to condition the hair. A common method of providing conditioning benefits to the hair is through the use of conditioning agents such as cationic surfactants and polymers, high melting point fatty compounds, low melting point oils, silicone compounds, and mixtures thereof.

However, there still exists the opportunity to increase the conditioning benefits delivered through the hair care compositions. There still exists a need for hair care compositions which provide improved silicone deposition and/or improved conditioning via friction reduction. Particularly, a need still exists to provide a hair care composition with enhanced benefits such as hair shine, softness, dry hair smoothness, hair strand alignment (e.g., minimized frizziness), and ease of combing. Also, a need still exists for a hair care composition that is effective for providing conditioning benefits to hair that is damaged by natural, environmental factors, as well as chemical hair treatments.

Patent application WO/2006138201 relates to a hair conditioning composition comprising silicone polymers containing quaternary groups. Example 7 of WO/2006138201 (see pages 29 to 30) is disclaimed from the claimed scope of the present invention. The relevant subject matter of WO/2006138201 also present in the priority document (US 60/691,175) filed on 16^{th} June 2005 is prior art pursuant to Article 54(3) of the European Patent Convention and thus relevant to the novelty of the present invention. Example 7 of WO/2006138201 can also be found in US 60/691,175. Example 7 of WO/2006138201 comprises *inter alia* a silicone quaternary polymer *1, HMW2220 silicone emulsion *5, behenyl trimethyl ammonium chloride *7, cetyl alcohol *12, stearyl alcohol * 13, deionised water.

### SUMMARY

The present invention provides unique hair care compositions. There has now been provided a hair care composition, comprising:
(a) an aminosilicone having a viscosity of from 1,000 centistokes to 1,000,000 centistokes, and less than 0.5% nitrogen by weight of the aminosilicone; and wherein the aminosilicone corresponds to the following formula:

   (R¹)ₐG₃₋ₐSi-(-OSiG₂)ₙ(-OSiG_{b}(R¹)_{2-b})ₘO-SiG₃₋ₐ(R¹)ₐ,

   wherein G comprises hydrogen, phenyl, OH, C₁-C₈ alkyl, or methyl and preferably is a methyl;
   a is an integer having a value from 1 to 3 and preferably is 1;
   b is 0, 1, or 2;
   n is a number from 0 to 1,999, and preferably is 400 to 600 or 1500 to 1700;
   m is an integer from 0 to 1,999 and preferably is 0; wherein the sum of n and m is a number from 1 to 2,000; and wherein a and m are not both 0;
   R¹ is a monovalent radical of formula CqH_{2q}L,
      wherein q is an integer from 2 to 8 and preferably is 3; and
      L comprises:

         -N(R²)CH₂-CH₂-N(R²)₂;

         -N(R²)₂;

         -N(R²)⁺₃A⁻;

         or

         -N(R²)CH₂-CH₂-NR²H⁺A⁻,

         wherein R² comprises hydrogen, phenyl, benzyl, a saturated hydrocarbon radical, or an alkyl radical containing from 1 to 20 carbon atoms and preferably is hydrogen or a methyl; and A⁻ denotes a halide ion;
(b) a silicone copolymer emulsion with an internal phase viscosity of greater than 120 x 10⁶ centistokes, as measured at 25°C; and
(c) optionally, a gel matrix comprising:
   i) a cationic surfactant;
   ii) a high melting point fatty compound; and
   iii) an aqueous carrier;
   and wherein the composition is a rinse-off conditioner, is a leave-on conditioner, or is not a shampoo product;

In accordance with another exemplary embodiment, there has now been provided a hair care composition comprising:
(a) an aminosilicone corresponding to the following formula:

   (R¹)ₐG₃₋ₐSi-(-OSiG₂)ₙ-(-OSiG_{b}(R¹)_{2-b})ₘ-O-SiG₃₋ₐ(R¹)ₐ,

   wherein G is selected from the group consisting of hydrogen, phenyl, OH, C₁-C₈ alkyl, and methyl;
   a is 0 or an integer having a value from 1 to 3;
   b is 0, 1, or 2;
   n is a number from 0 to 1,999;
   m is an integer from 0 to 1,999; wherein the sum of n and m is a number from 1 to 2,000 and wherein a and m are not both 0;
   R¹ is a monovalent radical of formula CqH_{2q}L,
      wherein q is an integer from 2 to 8; and
      L is selected from the group consisting of:

         -N(R²)CH₂-CH₂-N(R²)₂;

         -N(R²)₂;

         -N(R²)⁺₃A⁻;

         and

         -N(R²)CH₂-CH₂-NR²H⁺A⁻;

         wherein R² is selected from the group consisting of hydrogen, phenyl, benzyl, a saturated hydrocarbon radical, and an alkyl radical containing from 1 to 20 carbon atoms; and A⁻ denotes a halide ion; and
(b) a silicone copolymer emulsion with an internal phase viscosity of greater than about 120 x 10⁶ centistokes, as measured at 25°C;
   wherein the aminosilicone has less than about 0.5% nitrogen by weight of the aminosilicone.

The hair care compositions can be formulated into a variety of product forms, including shampoos, conditioners (both rinse-off and leave-on versions), styling products, and the like. In one embodiment, the hair care compositions include only hair conditioners and do not include shampoos or styling products.

Merchandising systems comprising hair care compositions of the present invention are also provided. In accordance with one exemplary embodiment, there has now been provided a merchandising system, including a hair care composition; and at least one of packaging for the composition and marketing material pertaining to the composition comprising indicia providing a communication to consumers related to dry hair, rough hair, frizzy hair, lusterless hair, hair breakage, hair fall or hair shed, and/or hair strength. The hair care composition comprises an aminosilicone and a silicone copolymer emulsion.

### DETAILED DESCRIPTION

The essential components of the hair care compositions are described below. Also included is a nonexclusive description of various optional and preferred components useful in embodiments of the present invention. While the specification concludes with claims that particularly point out and distinctly claim the invention, it is believed that the present invention will be better understood from the following description.

All percentages, parts, and ratios are based upon the total weight of the compositions of the present invention, unless otherwise specified. All such weights as they pertain to listed ingredients are based on the active level and, therefore do not include solvents or by-products that may be included in commercially available materials, unless otherwise specified. The term "weight percent" may be denoted as "wt.%" herein.

All molecular weights as used herein are weight average molecular weights expressed as grams/mole, unless otherwise specified.

The compositions and methods/processes of the present invention can comprise, consist of, and consist essentially of the essential elements and limitations of the invention described herein, as well as any of the additional or optional ingredients, components, steps, or limitations described herein.

### HAIR CARE COMPOSITIONS

### A. Aminosilicone

Compositions of the present invention include an aminosilicone. Aminosilicones, as provided herein, are silicones containing at least one primary amine, secondary amine, tertiary amine, or a quaternary ammonium group. Preferred aminosilicones may have less than about 0.5% nitrogen by weight of the aminosilicone, more preferably less than about 0.2%, more preferably still, less than about 0.1%. Higher levels of nitrogen (amine functional groups) in the amino silicone tend to result in less friction reduction, and consequently less conditioning benefit from the aminosilicone. It should be understood that in some product forms, higher levels of nitrogen are acceptable in accordance with the present invention.

Preferably, the amino silicones used in the present invention have a particle size of less than about 50µ once incorporated into the final composition. The particle size measurement is taken from dispersed droplets in the final composition. Particle size may be measured by means of a laser light scattering technique, using a Horiba model LA-910 Laser Scattering Particle Size Distribution Analyzer (Horiba Instruments, Inc.).

In one of the preferred embodiments, the aminosilicone has a viscosity of from about 1,000 cs (centistokes) to about 1,000,000 cs, more preferably from about 10,000 cs to about 700,000 cs, more preferably from about 50,000 cs to about 500,000 cs, and still more preferably from about 100,000 cs to about 400,000 cs. This embodiment may also comprises a low viscosity fluid, such as, for example, those materials described below in Section F.(1). The viscosity of aminosilicones discussed herein is measured at 25°C.

In another preferred embodiment, the aminosilicone has a viscosity of from about 1,000 cs to about 100,000 cs, more preferably from about 2,000 cs to about 50,000 cs, more preferably from about 4,000 cs to about 40,000 cs, and still more preferably from about 6,000 cs to about 30,000 cs.

The aminosilicone is contained in the composition of the present invention at a level by weight of from about 0.05% to about 20%, preferably from about 0.1% to about 10%, and more preferably from about 0.3% to about 5%.

Examples of preferred aminosilicones for use in embodiments of the subject invention include, but are not limited to, those which conform to the general formula (I):

(R¹)ₐG₃₋ₐ-Si-(-OSiG₂)ₙ-(-OSiG_{b}(R¹)_{2-b})ₘ-O-SiG₃₋ₐ(R¹)ₐ (I)

wherein G is hydrogen, phenyl, hydroxy, or C₁-C₈ alkyl, preferably methyl; a is 0 or an integer having a value from 1 to 3, preferably 1; b is 0, 1, or 2, preferably 1; wherein when a is 0, b is not 2; n is a number from 0 to 1,999; m is an integer from 0 to 1,999; the sum of n and m is a number from 1 to 2,000; a and m are not both 0; R¹ is a monovalent radical conforming to the general formula CqH_{2q}L, wherein q is an integer having a value from 2 to 8 and L is selected from the following groups:

-N(R²)CH₂-CH₂-N(R²)₂; -N(R²)₂; -N(R²)⁺₃A⁻; -N(R²)CH₂-CH₂-NR²H₂A⁻

wherein R² is hydrogen, phenyl, benzyl, or a saturated hydrocarbon radical, preferably an alkyl radical from about C₁ to about C₂₀; A is a halide ion.

Highly preferred aminosilicones are those corresponding to formula (I) wherein m=0, a=1, q=3, G=methyl, n is preferably from about 1500 to about 1700, more preferably about 1600; and L is -N(CH₃)₂ or -NH₂, more preferably -NH₂. Other highly preferred aminosilicones are those corresponding to formula (I) wherein m=0, a=1, q=3, G=methyl, n is preferably from about 400 to about 600, more preferably about 500; and L is -N(CH₃)₂ or -NH₂, more preferably-NH₂. These aminosilicones can be called as terminal aminosilicones, as one or both ends of the silicone chain are terminated by nitrogen containing group.

An exemplary amino silicone corresponding to formula (I) is the polymer known as "trimethylsilylamodimethicone", which is shown below in formula (II): 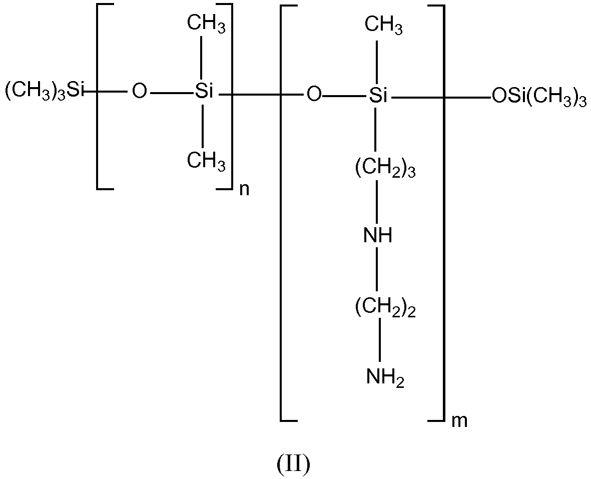 wherein n is a number from 1 to 1,999 and m is a number from 1 to 1,999.

### B. High Molecular Weight Silicone Copolymer Emulsion

Compositions of the present invention also comprise a silicone copolymer emulsion with an internal phase viscosity of greater than about 120 x 10⁶ cs. The silicone copolymer emulsion is present in an amount of from about 0.1% to about 15%, preferably from about 0.3% to about 10%, and more preferably from about 0.5% to about 5%, by weight of the composition.

The silicone copolymer emulsion has a viscosity at 25°C of greater than about 120 x 10⁶ cs, preferably greater than about 150 x 10⁶ cs, and preferably less than about 1000 x 10⁶ cs, more preferably less than about 500 x 10⁶ cs, and even more preferably less than about 300 x 10⁶ cs. To measure the internal phase viscosity of the silicone copolymer emulsion, one must first break the polymer from the emulsion. By way of example, the following procedure can be used to break the polymer from the emulsion: 1) add 10 grams of an emulsion sample to 15 milliliters of isopropyl alcohol; 2) mix well with a spatula; 3) decant the isopropyl alcohol; 4) add 10 milliliters of acetone and knead polymer with spatula; 5) decant the acetone; 6) place polymer in an aluminum container and flatten/dry with a paper towel; and 7) dry for two hours in an 80°C. The polymer can then be tested using any known rheometer, such as, for example, a CarriMed, Haake, or Monsanto rheometer, which operates in the dynamic shear mode. The internal phase viscosity values can be obtained by recording the dynamic viscosity (n') at a 9.900*10⁻³ Hz frequency point. The average particle size of the emulsions is preferably less than about 1 micron, more preferably less than about 0.7 micron.

The silicone copolymer emulsions of the present invention comprise a silicone copolymer, at least one surfactant, and water.

The silicone copolymer results from the addition reaction of the following two materials in the presence of a metal containing catalyst:
(a) a polysiloxane with reactive groups on both termini, represented by formula (III) 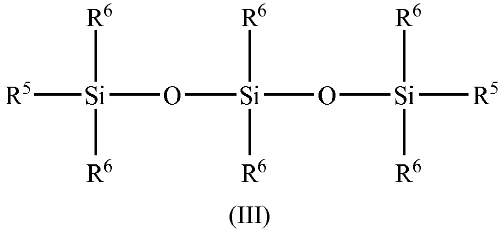 wherein:
   R⁵ is a group capable of reacting by chain addition reaction such as, for example, a hydrogen atom, an aliphatic group with ethylenic unsaturation (i.e., vinyl, allyl, or hexenyl), a hydroxyl group, an alkoxyl group (i.e., methoxy, ethoxy, or propoxy), an acetoxyl group, or an amino or alkylamino group; preferably, R⁵ is hydrogen or an aliphatic group with ethylenic unsaturation; more preferably, R⁵ is hydrogen;
   R⁶ is alkyl, cycloalkyl, aryl, or alkylaryl and may include additional functional groups such as ethers, hydroxyls, amines, carboxyls, thiols esters, and sulfonates; preferably, R⁶ is methyl. Optionally, a small mole percentage of the groups may be reactive groups as described above for R⁵, to produce a polymer which is substantially linear but with a small amount of branching. In this case, preferably the level of R⁶ groups equivalent to R⁵ groups is less than about 10% on a mole percentage basis, and more preferably less than about 2%;
   n is a whole number such that the polysiloxane of formula (III) has a viscosity of from about 1 cs to about 1 x 10⁶ cs;
   and,
(b) at least one silicone compound or non-silicone compound comprising at least one or at most two groups capable of reacting with the R₅ groups of the polysiloxane in formula (III); preferably, the reactive group is an aliphatic group with ethylenic unsaturation.

The metal containing catalysts used in the above described reactions are often specific to the particular reaction. Such catalysts are known in the art. Generally, they are materials containing metals such as platinum, rhodium, tin, titanium, copper, lead, etc.

The mixture used to form the emulsion also contains at least one surfactant. This can include non-ionic surfactants, cationic surfactants, anionic surfactants, alkylpolysaccharides, amphoteric surfactants, and the like. The above surfactants can be used individually or in combination.

An exemplary method of making the silicone copolymer emulsions described herein comprises the steps of 1) mixing materials (a) described above with material (b) described above, followed by mixing in an appropriate metal containing catalyst, such that material (b) is capable of reacting with material (a) in the presence of the metal containing catalyst; 2) further mixing in at least one surfactant and water; and 3) emulsifying the mixture. Methods of making such silicone copolymer emulsions are disclosed in U.S. Patent No. 6,013,682; PCT Application No. WO01/58986 A1; and European Patent Application No. EP0874017 A2.

### C. Gel Matrix

Some composition embodiments of the present invention comprise a gel matrix comprising (1) a cationic surfactant, (2) a high melting fatty compound, and (3) an aqueous carrier. The cationic surfactant, together with the high melting fatty compound, and an aqueous carrier, provides a gel matrix which is suitable for providing various conditioning benefits, especially slippery and slick feel on wet hair.

The cationic surfactant and the high melting point fatty compound are contained at a level such that the mole ratio of the cationic surfactant to the high melting point fatty compound is in the range of, preferably from about 1:1 to about 1:10, more preferably from about 1:2 to about 1:6 or from about 1:1 to about 1:4, in view of providing the above conditioning benefits especially slippery and slick feel on wet hair. Exemplary compositions of the present invention comprise, by weight of the composition, from about 60% to about 99%, preferably from about 70% to about 95%, and more preferably from about 80% to about 95% of a gel matrix including lamellar gel matrix, to which optional ingredients can be added (e.g., silicones).

### (1) Cationic Surfactant

The cationic surfactant is typically a mono-long alkyl quaternized ammonium salt having the following formula (IV) 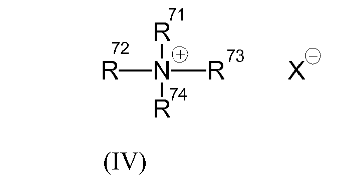 wherein one of R⁷¹, R⁷², R⁷³, and R⁷⁴ is selected from an aliphatic group of from about 16 to about 30 carbon atoms or an aromatic, alkoxy, polyoxyalkylene, alkylamido, hydroxyalkyl, aryl or alkylaryl group having up to about 30 carbon atoms; the remainder of R⁷¹, R⁷², R⁷³, and R⁷⁴ are independently selected from an aliphatic group of from about 1 carbon atom to about 8 carbon atoms or an aromatic, alkoxy, polyoxyalkylene, alkylamido, hydroxyalkyl, aryl, or alkylaryl group having up to about 8 carbon atoms; and X⁻ is a salt-forming anion such as those selected from halogen, (e.g., chloride, bromide), acetate, citrate, lactate, glycolate, phosphate, nitrate, sulfonate, sulfate, alkylsulfate, glutamate, and alkyl sulfonate radicals. The aliphatic groups can contain, in addition to carbon and hydrogen atoms, ether linkages, and other groups such as amino groups. The longer chain aliphatic groups, e.g., those of about 16 carbons, or higher, can be saturated or unsaturated. Preferably, one of R⁷¹, R⁷², R⁷³, and R⁷⁴ is selected from an alkyl group of from about 16 carbon atoms to about 30 carbon atoms, more preferably from about 18 to about 26 carbon atoms, still more preferably from about 22 carbon atoms; the remainder of R⁷¹, R⁷², R⁷³, and R⁷⁴ are independently selected from the group consisting of CH₃, C₂H₅, C₂H₄OH, CH₂C₆H₅, and mixtures thereof; and X is selected from the group consisting of Cl, Br, CH₃OSO₃, and mixtures thereof. It is believed that such mono-long alkyl quaternized ammonium salts can provide improved slippery and slick feel on wet hair, compared to multi-long alkyl quaternized ammonium salts. It is also believed that mono-long alkyl quaternized ammonium salts can provide improved hydrophobicity and smooth feel on dry hair, compared to amine or amine salt cationic surfactants.

Nonlimiting examples of such mono-long alkyl quaternized ammonium salt cationic surfactants include: behenyl trimethyl ammonium chloride available, for example, with tradename Genamine KDMP from Clariant, with tradename INCROQUAT TMC-80 from Croda and ECONOL TM22 from Sanyo Kasei; stearyl trimethyl ammonium chloride available, for example, with tradename CA-2450 from Nikko Chemicals; cetyl trimethyl ammonium chloride available, for example, with tradename CA-2350 from Nikko Chemicals; behenyltrimethylammonium methyl sulfate, available from FeiXiang; hydrogenated tallow alkyl trimethyl ammonium chloride; stearyl dimethyl benzyl ammonium chloride; and stearoyl amidopropyl dimethyl benzyl ammonium chloride.

Among them, more preferred cationic surfactants are those having a longer alkyl group, i.e., C₂₂ alkyl group. Such cationic surfactant includes, for example, behenyl trimethyl ammonium chloride and behenyltrimethylammonium methyl sulfate. It is believed that cationic surfactants having a longer alkyl group provide improved hydrophobicity on dry hair, compared to cationic surfactant having a shorter alkyl group. It is also believed that compared to cationic surfactants having a shorter alkyl group, cationic surfactants having a long alkyl group can provide improved hydrophobicity to the hair, especially to damaged hair, when combined with the polyol esters of the present invention. Alternatively, it is believed that cationic surfactant having an adequate length of alkyl group provides improved slippery and slick feel on wet hair, compared to a cationic surfactant having too long an alkyl group. Thus, it is believed that the selection of C₂₂ alkyl group among long alkyl groups provides balanced benefits between improved hydrophobicity on dry hair and improved slippery and slick feel on wet hair.

When present, the compositions of the present invention preferably comprise the cationic surfactant in amount of from about 0.1% to about 10%, more preferably from about 1% to about 8%, still more preferably from about 1.5% to about 5% by weight of the composition.

### (2) High Melting Point Fatty Compound

The high melting point fatty compounds useful herein have a melting point of about 25°C or higher, and are selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than about 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

The high melting point fatty compound can be included in the composition at a level of from about 0.1% to about 20%, preferably from about 1% to about 10%, still more preferably from about 2% to about 8%, by weight of the composition.

The fatty alcohols useful herein are those having from about 14 carbon atoms to about 30 carbon atoms, preferably from about 16 carbon atoms to about 22 carbon atoms. These fatty alcohols are saturated and can be straight or branched chain alcohols. Nonlimiting examples of fatty alcohols include cetyl alcohol, stearyl alcohol, behenyl alcohol, and mixtures thereof.

The fatty acids useful herein are those having from about 10 carbon atoms to about 30 carbon atoms, preferably from about 12 carbon atoms to about 22 carbon atoms, and more preferably from about 16 carbon atoms to about 22 carbon atoms. These fatty acids are saturated and can be straight or branched chain acids. Also included are diacids, triacids, and other multiple acids which meet the requirements herein. Also included herein are salts of these fatty acids. Nonlimiting examples of fatty acids include lauric acid, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, and mixtures thereof. Nonlimiting examples of fatty alcohol derivatives and fatty acid derivatives include materials such as methyl stearyl ether; the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through steareth-10, which are ethylene glycol ethers of steareth alcohol, wherein the numeric designation indicates the number of ethylene glycol moieties present; ceteareth-1 through ceteareth-10, which are the ethylene glycol ethers of ceteareth alcohol, i.e., a mixture of fatty alcohols containing predominantly cetyl and stearyl alcohol, wherein the numeric designation indicates the number of ethylene glycol moieties present; C₁-C₃₀ alkyl ethers of the ceteth, steareth, and ceteareth compounds just described; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, and mixtures thereof.

High melting point fatty compounds of a single compound of high purity are preferred. Single compounds of pure fatty alcohols selected from the group of pure cetyl alcohol, stearyl alcohol, and behenyl alcohol are highly preferred. By "pure" herein, what is meant is that the compound has a purity of at least about 90%, preferably at least about 95%. These single compounds of high purity provide good rinsability from the hair when the consumer rinses off the composition.

Commercially available high melting point fatty compounds useful herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan), various fatty acids having tradenames NEO-FAT available from Akzo (Chicago, Illinois USA), HYSTRENE available from Witco Corp. (Dublin, Ohio USA), and DERMA available from Vevy (Genova, Italy).

### (3) Aqueous Carrier

Carriers useful in the present invention include, for example, water and water solutions of lower alkyl alcohols and polyhydric alcohols. The lower alkyl alcohols useful herein are monohydric alcohols having from about 1 carbon atom to about 6 carbons, more preferably ethanol and isopropanol. The polyhydric alcohols useful herein include propylene glycol, hexylene glycol, glycerin, and propane diol.

Preferably, the aqueous carrier is substantially water. Deionized water is preferably used. Water from natural sources including mineral cations can also be used, depending on the desired characteristic of the product. Generally, the compositions of the present invention comprise from about 20% to about 95%, preferably from about 30% to about 92%, and more preferably from about 50% to about 90% water.

### D. Optional Components

### (1) Low Viscosity Fluid

The compositions of the present invention may optionally comprise a low viscosity fluid to be mixed with the aminosilicone described above. Exemplary low viscosity fluids have a viscosity of less than about 100 cs (although higher viscosity fluids may also be used). The low viscosity fluid, when combined with the aminosilicone, serves to reduce the viscosity of the aminosilicone in order to facilitate the processing into the complete composition.

Concentrations of the low viscosity fluid in the compositions of the present invention will vary primarily with the type and amount of fluid and aminosilicone employed. Preferred concentrations of the low viscosity fluid are from about 0.1% to about 20%, preferably from about 0.1% to about 10% by weight of the composition.

Preferred low viscosity fluids are partially or completely miscible with the amino silicone and are effective at reducing the viscosity of the blend. The low viscosity fluid or combination of fluids should be used in the composition so that there is the least amount of low viscosity fluid as possible. When the two materials are blended, the preferred viscosity range will be from about 500 cs to about 100,000 cs, more preferably from about 1,000 cs to about 50,000 cs.

The low viscosity fluid for the aminosilicone is suitable for topical application to human hair and scalp. The low viscosity fluid is organic, silicone-containing or fluorine-containing, volatile or non-volatile, polar or non-polar, provided that the fluid is partially or completely miscible with the amino silicone and yields a mixture with the above stated viscosity.

The low viscosity fluid preferably includes volatile, non-polar oils; non-volatile, relatively polar oils; non-volatile, non-polar oils; and non-volatile paraffinic hydrocarbon oils. The term "non-volatile" as used herein refers to materials which exhibit a vapor pressure of no more than about 0.2 mm Hg at 25°C at one atmosphere and/or to materials that have a boiling point at one atmosphere of at least about 300°C. The term "volatile" as used herein refers to all materials that are not "non-volatile" as previously defined herein. The phrase "relatively polar" as used herein means more polar than another material in terms of solubility parameter (e.g., the higher the solubility parameter the more polar the liquid). The term "non-polar" typically means that the material has a solubility parameter below about 6.5 (cal/cm³)^{0.5}.

### (a) Non-polar, Volatile Oils

Non-polar, volatile oils particularly useful in the present invention are selected from the group consisting of silicone oils, hydrocarbons, and mixtures thereof. Such non-polar, volatile oils are disclosed, for example, in Cosmetics, Science, and Technology, Vol. 1, 27-104. The non-polar, volatile oils useful in the present invention may be either saturated or unsaturated, have an aliphatic character and be straight or branched chained or contain alicyclic or aromatic rings. Examples of preferred non-polar, volatile hydrocarbons include polydecanes such as isododecane and isodecane (e.g., Permethyl-99A which is available from Presperse Inc.) and the C₇ - C₈ through C₁₂ - C₁₅ isoparaffins (such as the Isopar Series available from Exxon Chemicals). Other isoparaffins include, for example, Isozol series available from Nippon Petrochemicals Co., LTD, such as Isozol 200 (containing mainly C₈ isoparaffin), Isozol 300 (containing mainly C₁₂ isoparaffin), and Isozol 400 (containing mainly C₁₄ isoparaffin). Non-polar, volatile hydrocarbons, especially non-polar, volatile isoparaffins are highly preferred among a variety of low viscosity fluids, in view of reducing the viscosity of the aminosilicones and providing improved hair conditioning benefits such as reduced friction on dry hair.

Non-polar, volatile liquid silicone oils are disclosed in U.S. Patent No. 4,781,917. Additionally, a description of various volatile silicones materials is found in Todd et al., "Volatile Silicone Fluids for Cosmetics," Cosmetics and Toiletries, 91:27-32 (1976). Particularly preferred volatile silicone oils are selected from the group consisting of cyclic volatile silicones corresponding to the formula (V): 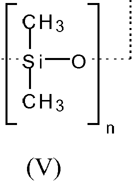 wherein n is from about 3 to about 7; and linear volatile silicones corresponding to the formula (VI):

(CH₃)₃ Si--O--[Si(CH₃)₂-O]ₘ --Si(CH₃)₃ (VI)

wherein m is from about 1 to about 7. Linear volatile silicones generally have a viscosity of less than about 5 cs at 25°C., whereas the cyclic silicones have viscosities of less than about 10 cs at 25°C. Highly preferred examples of volatile silicone oils include cyclomethicones of varying viscosities, e.g., Dow Corning 200, Dow Corning 244, Dow Corning 245, Dow Corning 344, and Dow Corning 345, (commercially available from Dow Corning Corp.); SF-1204 and SF-1202 Silicone Fluids (commercially available from G.E. Silicones), GE 7207 and 7158 (commercially available from General Electric Co.); and SWS-03314 (commercially available from SWS Silicones Corp.).

### (b) Relatively Polar, Non-volatile oils

The non-volatile oil is "relatively polar" as compared to the non-polar, volatile oil discussed above. Therefore, the non-volatile co-solvent is more polar (e.g., has a higher solubility parameter) than at least one of the non-polar, volatile oils. Relatively polar, non-volatile oils potentially useful in the present invention are disclosed, for example, in Cosmetics, Science, and Technology, Vol. 1, 27-104 edited by Balsam and Sagarin, 1972; U.S. Patent Nos. 4,202,879 and 4,816,261. Relatively polar, non-volatile oils useful in the present invention are preferably selected from the group consisting of silicone oils; hydrocarbon oils; fatty alcohols; fatty acids; esters of mono and dibasic carboxylic acids with mono and polyhydric alcohols; polyoxyethylenes; polyoxypropylenes; mixtures of polyoxyethylene and polyoxypropylene ethers of fatty alcohols; and mixtures thereof. The relatively polar, non-volatile co-solvents useful in the present invention may be either saturated or unsaturated, have an aliphatic character and be straight or branched chained or contain alicyclic or aromatic rings. More preferably, the relatively polar, non-volatile liquid co-solvents are selected from the group consisting of fatty alcohols having from about 12 carbon atoms to about 26 carbon atoms; fatty acids having from about 12 carbon atoms to about 26 carbon atoms; esters of monobasic carboxylic acids and alcohols having from about 14 carbon atoms to about 30 carbon atoms; esters of dibasic carboxylic acids and alcohols having from about 10 carbon atoms to about 30 carbon atoms; esters of polyhydric alcohols and carboxylic acids having from about 5 carbon atoms to about 26 carbon atoms; ethoxylated, propoxylated, and mixtures of ethoxylated and propoxylated ethers of fatty alcohols with from about 12 carbon atoms to about 26 carbon atoms and a degree of ethoxylation and propoxylation of below about 50; and mixtures thereof. More preferred are propoxylated ethers of C₁₄-C₁₈ fatty alcohols having a degree of propoxylation below about 50, esters of C₂-C₈ alcohols and C₁₂-C₂₆ carboxylic acids (e.g., ethyl myristate, isopropyl palmitate), esters of C₁₂-C₂₆ alcohols and benzoic acid (e.g., Finsolv TN supplied by Finetex), diesters of C₂-C₈ alcohols and adipic, sebacic, and phthalic acids (e.g., diisopropyl sebacate, diisopropyl adipate, di-n-butyl phthalate), polyhydric alcohol esters of C₆-C₂₆ carboxylic acids (e.g., propylene glycol dicaprate/dicaprylate, propylene glycol isostearate); and mixtures thereof. Even more preferred are branched-chain aliphatic fatty alcohols having from about 12 carbon atoms to about 26 carbon atoms. Even more preferred are isocetyl alcohol, octyldecanol, octyldodecanol and undecylpentadecanol; and even more preferred is octyldodecanol.

### (c) Non-polar, Non-volatile oils

In addition to the liquids discussed above, the low viscosity fluid may optionally include non-volatile, non-polar oils. Typical non-volatile, non-polar oils are disclosed, for example, in Cosmetics, Science, and Technology, Vol. 1, 27-104 edited by Balsam and Sagarin, 1972; U.S. Patent Nos. 4,202,879 and 4,816,261. The non-volatile oils useful in the present invention are essentially non-volatile polysiloxanes, paraffinic hydrocarbon oils, and mixtures thereof. The polysiloxanes useful in the present invention selected from the group consisting of polyalkylsiloxanes, polyarylsiloxanes, polyalkylarylsiloxanes, poly-ethersiloxane copolymers, and mixtures thereof. Examples of these include polydimethyl siloxanes having viscosities of from about 1 cs to about 100,000 cs at 25°C. Among the preferred non-volatile silicone emollients useful in the present compositions are the polydimethyl siloxanes having viscosities of from about 2 cs to about 400 cs at 25°C. Such polyalkylsiloxanes include the Viscasil series (sold by General Electric Co.) and the Dow Corning 200 series (sold by Dow Corning Corp.). Polyalkylarylsiloxanes include polymethylphenyl siloxanes having viscosities of from about 15 cs to about 65 cs at 25°C. These are available, for example, as SF 1075 methyl-phenyl fluid (sold by General Electric Co.) and 556 Cosmetic Grade Fluid (sold by Dow Corning Corp.). Useful polyethersiloxane copolymers include, for example, a polyoxyalkylene ether copolymer having a viscosity of from about 1200 cs to about 1500 cs at 25°C. Such a fluid is available as SF1066 organosilicone surfactant (sold by General Electric Co.). Polysiloxane ethylene glycol ether copolymers are preferred copolymers for use in the present compositions.

Non-volatile paraffinic hydrocarbon oils useful in the present invention include mineral oils and certain branched-chain hydrocarbons. Examples of these fluids are disclosed in U.S. Patent No. 5,019,375. Preferred mineral oils have the following properties:
(1) viscosity of from about 5 cs to about 70 cs at 40°C;
(2) density of from about 0.82 g/cm³ to about 0.89 g/cm³ at 25°C;
(3) flash point of from about 138°C to about 216°C; and
(4) carbon chain length of from about 14 carbon atoms to about 40 carbon atoms.
Preferred branched chain hydrocarbon oils have the following properties:
(1) density of from about 0.79 g/cm³ to about 0.89 g/cm³ at 20°C;
(2) boiling point greater than about 250°C; and
(3) flash point of from about 110°C to about 200°C.
Particularly preferred branched-chain hydrocarbons include Permethyl 103 A, which contains an average of about 24 carbon atoms; Permethyl 104A, which contains an average of about 68 carbon atoms; Permethyl 102A, which contains an average of about 20 carbon atoms; all of which may be purchased from Permethyl Corp.; and Ethylflo 364 which contains a mixture of 30 carbon atoms and 40 carbon atoms and may be purchased from Ethyl Corp.

Additional solvents useful herein are described in U.S. Patent No. 5,750,096.

### (2) Acid

The compositions of the present invention may further comprise an acid selected from the group consisting of L-glutamic acid, lactic acid, hydrochloric acid, malic acid, succinic acid, acetic acid, fumaric acid, L-glutamic acid hydrochloride, tartaric acid, citric acid, and mixtures thereof; preferably L-glutamic acid, lactic acid, citric acid, and mixtures thereof. The amines herein are preferably partially neutralized with any of the acids at a molar ratio of the amine to the acid of from about 1:0.3 to about 1:2, more preferably from about 1:0.4 to about 1:1.3.

### (3) Polysorbate

The compositions of the present invention may contain a polysorbate, in order to adjust rheology. Preferred polysorbates useful herein include polysorbate-20, polysorbate-21, polysorbate-40, polysorbate-60, and mixtures thereof. Highly preferred is polysorbate-20. The polysorbate can be contained in the composition at a level by weight of from about 0.01% to about 5%, preferably from about 0.05% to about 2%.

### (4) Polypropylene Glycol

Polypropylene glycols useful herein are those having a weight average molecular weight of from about 200 g/mol to about 100,000 g/mol, preferably from about 1,000 g/mol to about 60,000 g/mol. Without intending to be limited by theory, it is believed that the polypropylene glycol herein deposits onto, or is absorbed into hair to act as a moisturizer buffer, and/or provides one or more other desirable hair conditioning benefits.

The polypropylene glycol useful herein may be either water-soluble, water-insoluble, or may have a limited solubility in water, depending upon the degree of polymerization and whether other moieties are attached thereto. The desired solubility of the polypropylene glycol in water will depend in large part upon the form (e.g., leave-on, or rinse-off form) of the hair care composition. For example, in a rinse-off hair care composition, it is preferred that the polypropylene glycol herein has a solubility in water at 25°C of less than about 1 g/100 g water, more preferably a solubility in water of less than about 0.5 g/100 g water, and even more preferably a solubility in water of less than about 0.1 g/100 g water.

The polypropylene glycol can be included in the hair care compositions of the present invention at a level of from about 0.01% to about 10%, preferably from about 0.05% to about 6%, more preferably from about 0.1% to about 3% by weight of the composition.

### (5) Low Melting Point Oil

Low melting point oils useful herein are those having a melting point of less than 25°C. The low melting point oil useful herein is selected from the group consisting of: hydrocarbon having from about 10 carbon atoms to about 40 carbon atoms; unsaturated fatty alcohols having from about 10 carbon atoms to about 30 carbon atoms such as oleyl alcohol; unsaturated fatty acids having from about 10 carbon atoms to about 30 carbon atoms; fatty acid derivatives; fatty alcohol derivatives; ester oils such as pentaerythritol ester oils, trimethylol ester oils, citrate ester oils, and glyceryl ester oils; poly α-olefin oils; and mixtures thereof. Preferred low melting point oils herein are selected from the group consisting of: ester oils such as pentaerythritol ester oils, trimethylol ester oils, citrate ester oils, and glyceryl ester oils; poly α-olefin oils; and mixtures thereof.

Particularly useful pentaerythritol ester oils and trimethylol ester oils herein include pentaerythritol tetraisostearate, pentaerythritol tetraoleate, trimethylolpropane triisostearate, trimethylolpropane trioleate, and mixtures thereof. Such compounds are available from Kokyo Alcohol with tradenames KAKPTI, KAKTTI, and Shin-nihon Rika with tradenames PTO, ENUJERUBU TP3SO.

Particularly useful citrate ester oils herein include triisocetyl citrate with tradename CITMOL 316 available from Bernel, triisostearyl citrate with tradename PELEMOL TISC available from Phoenix, and trioctyldodecyl citrate with tradename CITMOL 320 available from Bernel.

Particularly useful glyceryl ester oils herein include triisostearin with tradename SUN ESPOL G-318 available from Taiyo Kagaku, triolein with tradename CITHROL GTO available from Croda Surfactants Ltd., trilinolein with tradename EFADERMA-F available from Vevy, or tradename EFA-GLYCERIDES from Brooks.

Particularly useful poly α-olefin oils herein include polydecenes with tradenames PURESYN 6 having a number average molecular weight of about 500 and PURESYN 100 having a number average molecular weight of about 3000 and PURESYN 300 having a number average molecular weight of about 6000 available from Exxon Mobil Co.

The low melting point oil can be included in the hair care compositions of the present invention at a level of from about 0.001% to about 10%, preferably up to about 5% by weight of the composition.

### (6) Cationic Polymer

Cationic polymers useful herein are those having an average molecular weight of at least about 5,000, typically from about 10,000 to about 10 million, preferably from about 100,000 to about 2 million.

Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol (made by hydrolysis of polyvinyl acetate), maleic anhydride, propylene glycol, and ethylene glycol. Other suitable cationic polymers useful herein include, for example, cationic celluloses, cationic starches, and cationic guar gums.

The cationic polymer can be included in the hair care compositions of the present invention at a level of from about 0.001% to about 10%, preferably up to about 5% by weight of the composition.

### (7) Polyethylene Glycol

Polyethylene glycol can also be used as an additional component. The polyethylene glycols useful herein correspond to the formula H(O-CH₂-CH₂)ₙOH, wherein n is the number of ethoxy units. Polyethylene glycols useful herein include PEG-2M wherein n has an average value of about 2,000 (PEG-2M is also known as Polyox WSR® N-10 from Union Carbide and as PEG-2,000); PEG-5M wherein n has an average value of about 5,000 (PEG-5M is also known as Polyox WSR® N-35 and as Polyox WSR® N-80, both from Union Carbide and as PEG-5,000 and Polyethylene Glycol 300,000); PEG-7M wherein n has an average value of about 7,000 (PEG-7M is also known as Polyox WSR® N-750 from Union Carbide); PEG-9M wherein n has an average value of about 9,000 (PEG-9M is also known as Polyox WSR® N-3333 from Union Carbide); and PEG-14M wherein n has an average value of about 14,000 (PEG-14M is also known as Polyox WSR® N-3000 from Union Carbide).

The polyethylene glycol can be included in the hair care compositions of the present invention at a level of from about 0.001% to about 10%, preferably up to about 5% by weight of the composition.

### E. Additional Components

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: other conditioning agents such as hydrolysed collagen with tradename Peptein 2000 available from Hormel; vitamin E with tradename Emix-d available from Eisai; panthenol available from Roche; panthenyl ethyl ether available from Roche; hydrolysed keratin, proteins, plant extracts, and nutrients; emollients such as PPG-3 myristyl ether with tradename Varonic APM available from Goldschmidt; Trimethyl pentanol hydroxyethyl ether; PPG-11 stearyl ether with tradename Varonic APS available from Goldschmidt; Stearyl heptanoate with tradename Tegosoft SH available from Goldschmidt; Lactil (mixture of Sodium lactate, Sodium PCA, Glycine, Fructose, Urea, Niacinamide, Inositol, Sodium Benzoate, and Lactic acid) available from Goldschmidt; Ethyl hexyl palmitate with tradename Saracos available from Nishin Seiyu and with tradename Tegosoft OP available from Goldschmidt; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents such as the thioglycolates; perfumes; sequestering agents, such as disodium ethylenediamine tetra-acetate; ultraviolet and infrared screening and absorbing agents such as octyl salicylate; and antidandruff agents such as zinc pyrithione and salicylic acid.

### ELASTICITY

It has been discovered that silicone blends useful in the present invention have a preferred tan δ value. Tan δ is a calculation related to the blended material's elasticity, and is equal to loss modulus (G") divided by storage modulus (G'). Loss modulus (G") and storage modulus (G') are measured by the following method:
Instrument: TA AR2000 or AR-G2 Rheometer, using the oscillation mode with a 40 mm diameter 2 degree AL cone and a gap of 57 µm.
Sample Preparation: scoop samples with spatula, and put the sample onto the measurement table of the rheometer without scrambling to avoid generation of bubbles.
Measurement Protocol: 1) equilibrate sample at a temperature of 26.7°C for 4 minutes; 2) begin oscillation at a frequency step of from 0.1 Hz to 10 Hz with the oscillation fixed at 5.0 Pa and the temperature at 26.7°C. The loss modulus and storage modulus readings are recorded and available via the rheometer instrumentation.

Exemplary silicone blends have tan δ values, as recorded at a frequency of 10 Hz and 26.7°C, from about 0.01 to about 5, preferably from about 0.05 to about 1, and more preferably from about 0.1 to about 0.5. Without being bound by theory, it is believed that the preferred tan δ value correlates to an in-use signal of end rinsing and/or clean feeling after rinsing, and consumers accordingly perceive compositions comprising these silicone blends to be superior in comparison to existing products.

### METHOD OF USE

The compositions of the present invention are used in conventional ways to provide conditioning and other benefits. Such method of use depends upon the type of composition employed but generally involves application of an effective amount of the product to the hair or scalp, which may then be rinsed from the hair or scalp or allowed to remain on the hair or scalp. "Effective amount" means an amount sufficient enough to provide a dry conditioning benefit. In general, from about 1g to about 50g is applied to the hair or scalp.

Preferably, the composition is applied to wet or damp hair prior to drying of the hair. The composition is distributed throughout the hair or scalp, typically by rubbing or massaging the hair or scalp. After such compositions are applied to the hair, the hair is dried and styled in accordance with the preference of the user. In the alternative, the composition is applied to dry hair, and the hair is then combed or styled in accordance with the preference of the user.

### MERCHANDISING SYSTEMS

Merchandising systems comprising hair care compositions of the present invention are also provided. In accordance with one exemplary embodiment, there has now been provided a merchandising system, including a hair care composition, and at least one of packaging for the composition and marketing material pertaining to the composition comprising indicia providing a communication to consumers related to dry hair, rough hair, frizzy hair, lusterless hair, hair breakage, hair fall or hair shed, and/or hair strength. The merchandising systems can include one or more of the hair care compositions described herein.

As used herein, "packaging" means a structure or material that is at least partially disposed on or about a hair care product when the product is presented to the public. "Primary packaging" means any container, including its closure, pump, cap, or other peripheral items, in which the hair care compositions is in direct contact. And "secondary packaging" means any additional materials that are associated with the primary packaging, such as, for example, a container such as a box or polymeric sleeve that at least partially surrounds, contains, or contacts the primary packaging.

As used herein, "advertisement material" means a tangible medium of expression, which by itself or with the aid of a peripheral device, makes known the existence of, or proclaims the quality or advantages of, an associated hair care composition.

As used herein, "indicia" means an identifying mark, including text and/or graphics.

### EXAMPLES

The compositions illustrated in the following examples exemplify specific embodiments of the compositions of the present invention, but are not intended to be limiting thereof.

The compositions illustrated in the following examples can be prepared by conventional formulation and mixing methods. All exemplified amounts are listed as weight percents and may exclude minor materials such as diluents, preservatives, color solutions, imagery ingredients, botanicals, and so forth, unless otherwise specified. In all examples, the term µm is synonymous with "micron."

**Examples 1-18 represent non-limiting examples of hair conditioner formulations.**

| Components | Ex.1 | Ex.2 | Ex.3 | Ex.4 | Ex.5 | Ex.6 | Ex.7 | Ex.8 | Ex. 9 |
|---|---|---|---|---|---|---|---|---|---|
| Stearamidopropyl Dimethylamine ¹ | 2.00 | 2.00 | 2.00 | 2.00 | 2.00 | 2.00 | 2.00 | 0 | 0 |
| Behenamidopropyl Dimethylamine ² | 0 | 0 | 0 | 0 | 0 | 0 | 0 | 2.30 | 2.30 |
| Glutamic Acid ³ | 0.64 | 0.64 | 0.64 | 0.64 | 0.64 | 0.64 | 0.64 | 0.64 | 0.64 |
| Cetyl alcohol ⁴ | 2.50 | 2.50 | 2.50 | 2.50 | 2.50 | 2.50 | 2.50 | 2.50 | 2.50 |
| Stearyl alcohol ⁵ | 4.50 | 4.50 | 4.50 | 4.50 | 4.50 | 4.50 | 4.50 | 4.50 | 4.50 |
| Amodimethicone ⁶ | 2.00 | 2.00 | 4.00 | 2.00 | 0 | 0 | 2.00 | 2.00 | 0 |
| Amodimethicone ⁷ | 0 | 0 | 0 | 0 | 3.00 | 2.70 | 0 | 0 | 3.00 |
| Dimethicone ⁸ | 3.00 | 0 | 6.00 | 0 | 0 | 0 | 0 | 0 | 0 |
| Dimethicone ⁹ | 0 | 3.00 | 0 | 3.00 | 0 | 0 | 0 | 3.00 | 0 |
| HMW2220 silicone emulsion ¹⁰ | 2.00 | 2.00 | 1.00 | 3.00 | 2.00 | 2.00 | 2.00 | 2.00 | 2.00 |
| PEG23/PPG6 Dimethicone¹¹ | 0 | 0 | 0 | 0 | 0 | 0.30 | 0 | 0 | 0 |
| Polysorbate-20 ¹⁴ | 0 | 0 | 0.20 | 0 | 0 | 0 | 0 | 0 | 0 |
| PPG-34 ¹⁵ | 0.50 | 0 | 0 | 0 | 0 | 0 | 0 | 0 | 0 |
| Polyalphaolefin ¹⁶ | 0 | 0.50 | 0 | 0 | 0 | 0 | 0 | 0 | 0 |
| Benzyl alcohol | 0.40 | 0.40 | 0.40 | 0.40 | 0.40 | 0.40 | 0.40 | 0.40 | 0.40 |
| Methylchloro isothiaz olinone/ Methylisothiazolinone ¹⁷ | 0.0005 | 0.0005 | 0.0005 | 0.0005 | 0.0005 | 0.0005 | 0.0005 | 0.0005 | 0.0005 |
| Perfume | 0.50 | 0.50 | 0.50 | 0.35 | 0.35 | 0.35 | 0.35 | 0.50 | 0.50 |
| NaOH | 0.014 | 0.014 | 0.014 | 0.014 | 0.014 | 0.014 | 0.014 | 0.014 | 0.014 |
| Panthenol ¹⁸ | 0.05 | 0.05 | 0 | 0.05 | 0.05 | 0.05 | 0.05 | 0 | 0 |
| Panthenyl ethyl ether 19 | 0.05 | 0.05 | 0 | 0.05 | 0.05 | 0.05 | 0.05 | 0 | 0 |
| Hydrolyzed collagen 20 | 0.01 | 0 | 0 | 0 | 0 | 0 | 0 | 0 | 0 |
| Vitamin E ²¹ | 0.01 | 0 | 0 | 0 | 0 | 0 | 0 | 0 | 0 |
| Octyl methoxycinnamate | 0.09 | 0 | 0 | 0 | 0 | 0 | 0 | 0 | 0 |
| Benzophenone-3 | 0.09 | | 0 | 0 | | 0 | 0 | 0 | 0 |
| Disodium EDTA | 0.127 | 0.127 | 0.127 | 0.127 | 0.127 | 0.127 | 0.127 | 0.127 | 0.127 |
| Deionized Water | q.s. | q.s. | q.s. | q.s. | q.s. | q.s. | q.s. | q.s. | q.s. |

| Components | Ex.10 | Ex.11 | Ex.12 | Ex.13 | Ex.14 | Ex.15 | Ex.16 | Ex.17 | Ex.18 |
|---|---|---|---|---|---|---|---|---|---|
| Behenyl trimethyl ammonium chloride ¹² | 2.25 | 2.25 | 2.25 | 2.25 | 2.25 | 2.25 | 0 | 0 | 0 |
| Behenyl trimethyl ammonium, methyl sulfate ¹³ | 0 | 0 | 0 | 0 | 0 | 0 | 1.80 | 1.80 | 1.80 |
| Isopropyl alcohol | 0.60 | 0.60 | 0.60 | 0.60 | 0.60 | 0.60 | 0.45 | 0.45 | 0.45 |
| Cetyl alcohol ⁴ | 1.86 | 1.86 | 1.86 | 1.86 | 1.86 | 1.86 | 1.48 | 1.48 | 1.48 |
| Stearyl alcohol ⁵ | 4.64 | 4.64 | 4.64 | 4.64 | 4.64 | 4.64 | 3.71 | 3.71 | 3.71 |
| Amodimethicone ⁶ | 2.00 | 4.00 | 0 | 0 | 0 | 2.00 | 2.00 | 0 | 0 |
| Amodimethicone ⁷ | 0 | 0 | 3.00 | 2.70 | 3.00 | 3.00 | 0 | 2.00 | 2.70 |
| Dimethicone ⁸ | 3.0 | 0 | 0 | 0 | 0 | 0 | 3.0 | 0 | 0 |
| Dimethicone ⁹ | 0 | 6.00 | 0 | 0 | 0 | 0 | 0 | 3.00 | 0 |
| HMW2220 silicone emulsion ¹⁰ | 2.00 | 1.00 | 2.00 | 2.00 | 2.00 | 2.00 | 2.00 | 2.00 | 2.00 |
| PEG23/PPG6 Dimethicone ¹¹ | 0 | 0 | 0 | 0.30 | 0 | 0 | 0 | 0 | 0.30 |
| Polysorbate-20 ¹⁴ | 0.20 | 0 | 0 | 0 | 0.20 | 0 | 0 | 0 | 0 |
| PPG-34 ¹⁵ | 0.50 | 0 | 0 | 0 | 0 | 0.50 | 0 | 0 | 0 |
| Polyalphaolefin ¹⁶ | 0 | 0.50 | 0 | 0 | 0 | 0 | 0 | 0 | 0 |
| Benzyl alcohol | 0.40 | 0.40 | 0.40 | 0.40 | 0.40 | 0.40 | 0.40 | 0.40 | 0.40 |
| Methylchloro isothiaz olinone/ Methylisothiazolinone 17 | 0.0005 | 0.0005 | 0.0005 | 0.0005 | 0.0005 | 0.0005 | 0.0005 | 0.0005 | 0.0005 |
| Perfume | 0.50 | 0.50 | 0.40 | 0.40 | 0.40 | 0.40 | 0.40 | 0.40 | 0.40 |
| NaOH | 0.014 | 0.014 | 0.014 | 0.014 | 0.014 | 0.014 | 0 | 0 | 0 |
| Panthenol ¹⁸ | 0.05 | 0.05 | 0.03 | 0.03 | 0.03 | 0.03 | 0.03 | 0.03 | 0.03 |
| Panthenyl ethyl ether 19 | 0.05 | 0.05 | 0.03 | 0.03 | 0.03 | 0.03 | 0.03 | 0.03 | 0.03 |
| Hydrolyzed collagen 20 | 0.01 | 0 | 0 | 0 | 0.01 | 0.01 | 0 | | |
| Vitamin E ²¹ | 0.01 | 0 | 0 | 0 | 0.01 | 0.01 | 0 | | |
| Octyl methoxycinnamate | 0 | 0 | 0 | 0 | 0 | 0 | 0 | | |
| Benzophenone-3 | 0.09 | | 0 | 0 | 0.09 | 0.09 | 0 | | |
| Disodium EDTA | 0.127 | 0.127 | 0.127 | 0.127 | 0.127 | 0.127 | 0.127 | 0.127 | 0.127 |
| Deionized Water | q.s. | q.s. | q.s. | q.s. | q.s. | q.s. | q.s. | q.s. | q.s. |

### Definitions of Components for Examples 1-18

- 1: Stearamidopropyl dimethylamine: Lexamine™ S-13 available from Inolex
- 2: Behenamidopropyl Dimethylamine (IncromineBB™) available from Croda
- 3: Glutamic acid: available from Ajinomoto
- 4: Cetyl alcohol: Konol™ series available from Shin Nihon Rika
- 5: Stearyl alcohol: Konol™ series available from Shin Nihon Rika
- 6: Terminal aminosilicone, AP type, available from General Electric; viscosity range from 220,000 cs to 45,000 cs
- 7: Terminal aminosilicone, AP type, available from General Electric; viscosity 10,000 cs
- 8: TSF-451-20A (20cs straight oil), available from General Electric
- 9: Dow Corning™ 200 Fluid, 10 cs
- 10: HMW2220 silicone emulsion, approx 60% silicone, available from Dow Corning
- 11: PEG23/PPG6 Dimethicone: Silsoft™475 available from GE Toshiba
- 12: Behenyl trimethyl ammonium chloride/Isopropyl alcohol: Genamin™ KDMP available from Clariant
- 13: Behenyltrimethylammonium methyl sulfate , available from FeiXiang
- 14: Polysorbate-20: Glycosperse™ L-20K available from Lonza Inc.
- 15: PPG-34: New Pol PP-2000 available from Sanyo Kasei
- 16: Polyalphaolefin: PureSyn™ 100 available from Exxon Mobil Chemical Company
- 17: Methylchloroisothiazolinone/Methylisothiazolinone: Kathon™ CG available from Rohm & Haas
- 18: Panthenol: Available from Roche
- 19: Panthenyl ethyl ether: Available from Roche
- 20: Hydrolyzed collagen: Peptein™ 2000 available from Hormel
- 21: Vitamin E: Emix-d available from Eisai

Examples 1-18 can be prepared by any conventional method well known in the art. They are suitably made as follows: deionized water is heated to 85°C and cationic surfactants and high melting point fatty compounds are mixed in. If necessary, cationic surfactants and fatty alcohols can be pre-melted at 85°C before addition to the water. The water is maintained at a temperature of about 85°C until the components are homogenized, and no solids are observed. The mixture is then cooled to about 55°C and maintained at this temperature, to form a gel matrix. Aminosilicones, or a blend of aminosilicones and a low viscosity fluid, or an aqueous dispersion of an aminosilicione are added to the gel matrix. The high viscosity silicone copolymer emulsions are also added. When included, poly alpha-olefin oils, polypropylene glycols, and/or polysorbates are also added to the gel matrix. When included, other additional components such as perfumes and preservatives are added with agitation. The gel matrix is maintained at about 50°C during this time with constant stirring to assure homogenization. After it is homogenized, it is cooled to room temperature. A triblender and/or mill can be used in each step, if necessary to disperse the materials.

## Claims

1. A hair care composition, comprising:
(a) an aminosilicone having a viscosity of from 1,000 centistokes to 1,000,000 centistokes, and less than 0.5% nitrogen by weight of the aminosilicone; and wherein the aminosilicone corresponds to the following formula:
(R¹)ₐG₃₋ₐ-Si-(-OSiG₂)ₙ-(-OSiG_{b}(R)_{2-b})ₘ-O-SiG₃₋ₐ(R¹)ₐ,
wherein G comprises hydrogen, phenyl, OH, C₁-C₈ alkyl, or methyl and preferably is a methyl;
a is an integer having a value from 1 to 3 and preferably is 1;
b is 0, 1, or 2;
n is a number from 0 to 1,999, and preferably is 400 to 600 or 1500 to 1700;
m is an integer from 0 to 1,999 and preferably is 0; wherein the sum of n and m is a number from 1 to 2,000; and wherein a and m are not both 0;
R¹ is a monovalent radical of formula CqH_{2q}L,
wherein q is an integer from 2 to 8 and preferably is 3; and
L comprises:
-N(R²)CH₂-CH₂-N(R²)₂;
-N(R²)₂;
-N(R²)⁺₃A⁻;
or
-N(R²)CH₂-CH₂-NR²H⁺A⁻,
wherein R² comprises hydrogen, phenyl, benzyl, a saturated hydrocarbon radical, or an alkyl radical containing from 1 to 20 carbon atoms and preferably is hydrogen or a methyl; and A⁻ denotes a halide ion;
(b) a silicone copolymer emulsion with an internal phase viscosity of greater than 120 x 10⁶ centistokes, as measured at 25°C; and
(c) optionally, a gel matrix comprising:
i) a cationic surfactant;
ii) a high melting point fatty compound; and
iii) an aqueous carrier;
and wherein the composition is a rinse-off conditioner, is a leave-on conditioner, or is not a shampoo product.

2. A composition according to any of the preceding claims, wherein the aminosilicone has less than 0.3% nitrogen, and preferably less than 0.2% nitrogen, by weight of the aminosilicone.

3. A composition according to any of the preceding claims, wherein the aminosilicone comprises trimethylsilyamodimethicone.

4. A composition according to any of the preceding claims, wherein the aminosilicone has a viscosity of from 100,000 centistokes to 400,000 centistokes.

5. A composition according to claim 4, wherein the composition further comprises a low viscosity fluid having a viscosity of less than 100 centistokes, wherein the low viscosity fluid is included to manage the viscosity of the aminosilicone.

6. A composition according to any of the preceding claims, wherein the aminosilicone has a viscosity of from 6,000 centistokes to 30,000 centistokes.

7. A composition according to any of the preceding claims, wherein the silicone copolymer emulsion has an internal phase viscosity of greater than 150 x 10⁶ centistokes, as measured at 25°C.

8. A composition according to any of the preceding claims, wherein the silicone copolymer emulsion comprises a silicone copolymer, at least one surfactant, and water, wherein the silicone copolymer comprises a polysiloxane having reactive groups on both termini, and corresponding to the following formula: 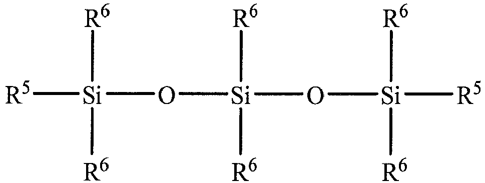 wherein R⁵ comprises a hydrogen atom, an aliphatic group with ethylenic unsaturation, a hydroxyl group, an alkoxyl group, an acetoxyl group, an amino or alkylamino group, and mixtures thereof; and R⁶ comprises an alkyl, a cycloalkyl, an aryl, analkylaryl, ethers, hydroxyls, amines, carboxyls, thiols esters, sulfonates, or mixtures thereof.

9. A composition according to any of the preceding claims, wherein a combination of the aminosilicone and the silicone copolymer emulsion have a tan δ value from 0.01 to 5, preferably from 0.05 to 1, and more preferably from 0.1 to 0.5.

10. A composition according to any of the preceding claims, wherein a combination of the aminosilicone and the silicone copolymer form an emulsion.

11. A composition according to any of the preceding claims, wherein m=0, a=1, q=3, G=methyl, n is preferably from 1500 to 1700, more preferably 1600; and L is -N(CH₃)₂ or -NH₂, more preferably -NH₂.

12. A composition according to any of the preceding claims, wherein m=0, a=1, q=3, G=methyl, n is preferably from 400 to 600, more preferably 500; and L is -N(CH₃)₂ or-NH₂, more preferably -NH₂.

13. A composition according to any of claims 11 to 12, wherein the aminosilicone is a terminal aminosilicone, as one or both ends of the silicone chain are terminated by nitrogen containing group.

## Patentansprüche

1. Haarpflegezusammensetzung, die Folgendes umfasst:
(a) ein Aminosilicon mit einer Viskosität zwischen 1.000 mm²/s und 1.000.000 mm²/s (1.000 Centistokes bis 1.000.000 Centistokes) und weniger als 0,5 Gew.-% Stickstoff bezogen auf das Gewicht des Aminosilicons; und wobei das Aminosilicon der folgenden Formel entspricht:
(R¹)ₐG₃₋ₐ-Si-(-OSiG₂)ₙ-(-OSiG_{b}(R¹)_{2-b})ₘ-O-SiG₃₋ₐ(R¹)ₐ,
worin G Wasserstoff, Phenyl, OH, C₁-C₈-Alkyl oder Methyl umfasst und vorzugsweise ein Methyl ist;
a eine ganze Zahl mit einem Wert von 1 bis 3 und vorzugsweise 1 ist;
b 0, 1 oder 2 ist;
n eine Zahl von 0 bis 1.999 und vorzugsweise 400 bis 600 oder 1500 bis 1700 ist;
m eine ganze Zahl von 0 bis 1.999 und vorzugsweise 0 ist; wobei die Summe aus n und m eine Zahl von 1 bis 2.000 ist; und wobei a und m nicht beide 0 sind;
R¹ ein einwertiger Rest der Formel C_{q}H_{2q}L ist,
worin q eine ganze Zahl von 2 bis 8 und vorzugsweise 3 ist; und L Folgendes umfasst:
- N(R²)CH₂-CH₂-N(R²)₂;
-N(R²)₂;
- N(R²)⁺₃A⁻;
oder
- (R²)CH₂-CH₂-NR²H⁺A⁻,
worin R² Wasserstoff, Phenyl, Benzyl, einen gesättigten Kohlenwasserstoffrest oder einen Alkylrest, der 1 bis 20 Kohlenstoffatome enthält, umfasst und vorzugsweise Wasserstoff oder ein Methyl ist; und A⁻ ein Halogenidion bezeichnet;
(b) eine Siliconcopolymeremulsion mit einer mit einer Viskosität der internen Phase von mehr als 120 x 10⁶ mm²/s (120 x 10⁶ Centistokes), gemessen bei 25 °C; und
(c) wahlweise eine Gelmatrix, die Folgendes umfasst:
i) ein kationisches Tensid;
ii) einen Fettstoff mit hohem Schmelzpunkt; und
iii) einen wässrigen Träger;
und wobei es sich bei der Zusammensetzung um eine Haarspülung zum Ausspülen, eine Haarspülung, die im Haar verbleibt, oder nicht um ein Shampooprodukt handelt.

2. Zusammensetzung nach einem der vorstehenden Ansprüche, wobei das Aminosilicon weniger als 0,3 Gew.-% Stickstoff und vorzugsweise weniger als 0,2 Gew.-% Stickstoff bezogen auf das Gewicht des Aminosilicons aufweist.

3. Zusammensetzung nach einem der vorstehenden Ansprüche, wobei das Aminosilicon Trimethylsilylamodimethicon umfasst.

4. Zusammensetzung nach einem der vorstehenden Ansprüche, wobei das Aminosilicon eine Viskosität von 100.000 mm²/s bis 400.000 mm²/s (100.000 Centistokes bis 400.000 Centistokes) aufweist.

5. Zusammensetzung nach Anspruch 4, wobei die Zusammensetzung ferner ein niedrigviskoses Fluid mit einer Viskosität von weniger als 100 mm²/s (100 Centistokes) umfasst, wobei das niedrigviskose Fluid enthalten ist, um die Viskosität des Aminosilicons zu regeln.

6. Zusammensetzung nach einem der vorstehenden Ansprüche, wobei das Aminosilicon eine Viskosität von 6.000 mm²/s bis 30.000 mm²/s (6.000 Centistokes bis 30.000 Centistokes) aufweist.

7. Zusammensetzung nach einem der vorstehenden Ansprüche, wobei die Siliconcopolymeremulsion eine Viskosität der internen Phase von mehr als 150 x 10⁶ mm²/s (150 x 10⁶ Centistokes), gemessen bei 25 °C, aufweist.

8. Zusammensetzung nach einem der vorstehenden Ansprüche, wobei die Siliconcopolymeremulsion ein Siliconcopolymer, mindestens ein Tensid, und Wasser umfasst,
wobei das Siliconcopolymer ein Polysiloxan mit reaktiven Gruppen an beiden Enden umfasst und der folgenden Formel entspricht: 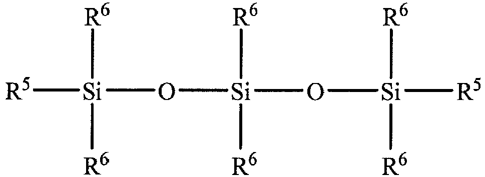 worin R⁵ ein Wasserstoffatom, eine aliphatische Gruppe mit ethylenischer Ungesättigtheit, eine Hydroxylgruppe, eine Alkoxylgruppe, eine Acetoxylgruppe, eine Amino- oder Alkylaminogruppe und Mischungen davon umfasst; und R⁶ ein Alkyl, ein Cycloalkyl, ein Aryl, Analkylaryl, Ether, Hydroxyle, Amine, Carboxyle, Thiolester, Sulfonate oder Mischungen davon umfasst.

9. Zusammensetzung nach einem der vorstehenden Ansprüche, wobei eine Kombination des Aminosilicons und der Siliconcopolymeremulsion einen Tan-δ-Wert von 0,01 bis 5, vorzugsweise von 0,05 bis 1, und stärker bevorzugt von 0,1 bis 0,5, aufweist.

10. Zusammensetzung nach einem der vorstehenden Ansprüche, wobei eine Kombination des Aminosilicons und des Siliconcopolymers eine Emulsion bilden.

11. Zusammensetzung nach einem der vorstehenden Ansprüche, worin m=0, a=1, q=3, G=Methyl, n vorzugsweise von 1500 bis 1700, weiter bevorzugt 1600, ist; und L -N(CH₃)₂ oder -NH₂, weiter bevorzugt -NH₂, ist.

12. Zusammensetzung nach einem der vorstehenden Ansprüche, worin m=0, a=1, q=3, G=Methyl, n vorzugsweise von 400 bis 600, weiter bevorzugt 500, ist; und L -N(CH₃)₂ oder -NH₂, weiter bevorzugt -NH₂, ist.

13. Zusammensetzung nach einem der Ansprüche 11 bis 12, wobei es sich bei dem Aminosilicon um ein endständiges Aminosilicon handelt, da eines oder beide Enden der Siliconkette von einer Stickstoff enthaltenden Gruppe begrenzt werden.

## Revendications

1. Composition pour soins capillaires comprenant :
(a) une aminosilicone ayant une viscosité allant de 1000 mm²/s à 1 000 000 mm²/s (1000 centistokes à 1 000 000 centistokes), et moins de 0,5 % d'azote en poids de l'aminosilicone ; et dans laquelle l'aminosilicone correspond à la formule suivante :
(R¹)ₐG₃₋ₐ-Si-(-OSiG₂)ₙ-(-OSiG_{b}(R¹)_{2-b})ₘ-O-SiG₃₋ₐ(R¹)ₐ,
dans laquelle G comprend hydrogène, phényle, OH, alkyle en C₁ à C₈, ou méthyle et est de préférence un méthyle ;
a est un nombre entier ayant une valeur de 1 à 3 et est de préférence 1 ;
b est 0, 1, ou 2 ;
n est un nombre de 0 à 1999, et va de préférence de 400 à 600 ou de 1500 à 1700 ;
m est un nombre entier allant de 0 à 1999 et est de préférence 0 ; dans laquelle la somme de n et de m est un nombre de 1 à 2000 ; et dans laquelle a et m ne sont pas l'un et l'autre 0 ;
R¹ est un radical monovalent de formule C_{q}H_{2q}L,
dans laquelle q est un nombre entier allant de 2 à 8 et est de préférence 3 ; et L comprend :
-N(R²)CH₂-CH₂-N(R²)₂ ;
-N(R²)₂ ;
-N(R²)+3A⁻ ;
ou
-N(R²)CH₂-CH₂-NR²H⁺A-,
dans laquelle R² comprend hydrogène, phényle, benzyle, un radical hydrocarbure saturé, ou un radical alkyle contenant de 1 à 20 atomes de carbone et est de préférence hydrogène ou un méthyle ; et A⁻ désigne un ion halogénure ;
(b) une émulsion de copolymère de silicone avec une viscosité de phase interne supérieure à 120 x 10⁶ mm²/s (120 x 10⁶ centistokes), telle que mesurée à 25 °C ; et
(c) facultativement, une matrice de gel comprenant :
i) un agent tensioactif cationique ;
ii) un composé gras à point de fusion élevé ; et
iii) un véhicule aqueux ;
et où la composition est un conditionneur à éliminer par rinçage, un conditionneur à conserver, ou n'est pas un produit de shampooing.

2. Composition selon l'une quelconque des revendications précédentes, dans laquelle l'aminosilicone possède moins de 0,3 % d'azote, et de préférence moins de 0,2 % d'azote, en poids de l'aminosilicone.

3. Composition selon l'une quelconque des revendications précédentes, dans laquelle l'aminosilicone comprend de la triméthylsilyamodiméthicone.

4. Composition selon l'une quelconque des revendications précédentes, dans laquelle l'aminosilicone a une viscosité allant de 100 000 mm²/s à 400 000 mm²/s (100 000 centistokes à 400 000 centistokes).

5. Composition selon la revendication 4, où la composition comprend en outre un fluide de basse viscosité ayant une viscosité inférieure à 100 mm²/s (100 centistokes), dans laquelle le fluide de basse viscosité est inclus pour gérer la viscosité de l'aminosilicone.

6. Composition selon l'une quelconque des revendications précédentes, dans laquelle l'aminosilicone a une viscosité allant de 6000 mm²/s à 30 000 mm²/s (6000 centistokes à 30 000 centistokes).

7. Composition selon l'une quelconque des revendications précédentes, dans laquelle l'émulsion de copolymère de silicone a une viscosité de phase interne supérieure à 150 x 10⁶ mm²/s (150 x 10⁶ centistokes), telle que mesurée à 25 °C.

8. Composition selon l'une quelconque des revendications précédentes, dans laquelle l'émulsion de copolymère de silicone comprend un copolymère de silicone, au moins un agent tensioactif, et de l'eau,
dans laquelle le copolymère de silicone comprend un polysiloxane comportant des groupes réactifs sur l'une et l'autre des terminaisons, et correspondant à la formule suivante : 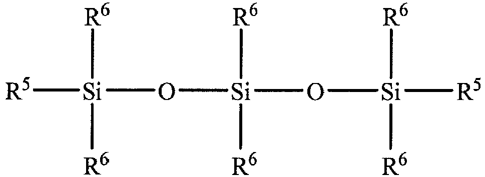 dans laquelle R⁵ comprend un atome d'hydrogène, un groupe aliphatique avec une insaturation éthylénique, un groupe hydroxyle, un groupe alcoxyle, un groupe acétoxyle, un groupe amino ou alkylamino, et des mélanges de ceux-ci ; et R⁶ comprend un alkyle, un cycloalkyle, un aryle, un alkylaryle, des éthers, des hydroxyles, des amines, des carboxyles, des esters de thiol, des sulfonates, ou des mélanges de ceux-ci.

9. Composition selon l'une quelconque des revendications précédentes, dans laquelle une combinaison de l'aminosilicone et de l'émulsion de copolymère de silicone a une valeur tan δ allant de 0,01 à 5, de préférence de 0,05 à 1, et plus préférablement de 0,1 à 0,5.

10. Composition selon l'une quelconque des revendications précédentes, dans laquelle une combinaison de l'aminosilicone et du copolymère de silicone forme une émulsion.

11. Composition selon l'une quelconque des revendications précédentes, dans laquelle m=0, a=1, q=3, G=méthyle, n va de préférence de 1500 à 1700, plus préférablement, 1600 ; et L est -N(CH₃)₂ ou -NH₂, plus préférablement -NH₂.

12. Composition selon l'une quelconque des revendications précédentes, dans laquelle m=0, a=1, q=3, G=méthyle, n va de préférence de 400 à 600, plus préférablement, 500 ; et L est -N(CH₃)₂ ou -NH₂, plus préférablement -NH₂.

13. Composition selon l'une quelconque des revendications 11 à 12, dans laquelle l'aminosilicone est une aminosilicone terminale, car l'une et/ou l'autre des extrémités de la chaîne silicone sont terminées par un groupe contenant de l'azote.
